# EUROPEAN PATENT APPLICATION

(11) **EP 4 198 821 A1**
(43) Date of publication of application: **21.06.2023**
(21) Application number: 21896886.5
(22) Date of filing: 19.11.2021
(51) Int. Cl.: G06K 9/62, G06N 3/04

(54) **GRAPH NEURAL NETWORK-BASED CLINICAL OMICS DATA PROCESSING METHOD AND APPARATUS, DEVICE, AND MEDIUM**

(30) Priority: 30.11.2020 CN 202011379315
(71) Applicant: Tencent Technology (Shenzhen) Company Limited, Shenzhen, Guangdong 518057 (CN)
(72) Inventor: XING, Xiaohan, Shenzhen, Guangdong 518057 (CN); YANG, Fan, Shenzhen, Guangdong 518057 (CN); YAO, Jianhua, Shenzhen, Guangdong 518057 (CN)
(74) Representative: AWA Sweden AB
(86) International application number: PCT/CN2021/131652
(87) International publication number: WO 2022/111385

(57) **Abstract**

A graph neural network-based clinical omics data processing method and apparatus, a device, and a medium, relating to the technical fields of medical treatment, artificial intelligence, cloud data and the like. The method comprises: obtaining first omics data of a target object, and extracting at least two first omics features from the first omics data (S101); determining a first correlation between different omics features in the at least two first omics features (S102); constructing a first graph structure corresponding to the first omics data according to the at least two first omics features and the first correlation, wherein the first graph structure comprises at least two nodes, and each node represents one first omics feature in the first omics data, the first graph structure at least comprises a connection edge connecting the at least two nodes, and the two nodes connected by the connection edge correspond to the first correlation (S103); according to the first graph structure, obtaining the node feature of each node in the first graph structure by means of a first graph neural network, wherein the node feature has at least one dimension (S104); and according to the node feature of each node, performing medical analysis on the target object to obtain a medical analysis result corresponding to each dimension in the at least one dimension, wherein the medical analysis comprises performing disease diagnosis, disease typing, and survival prediction on the target object, and the medical analysis result comprises a probability that the target object corresponding to each dimension has a disease, a probability that the disease of the target object corresponding to each dimension is a certain disease category, and a survival probability of the target object corresponding to each dimension (S105).

## Description

### RELATED APPLICATION

This application claims priority to Chinese Patent Application No. 202011379315.3, entitled "OMICS DATA PROCESSING METHOD AND APPARATUS BASED ON GRAPH NEURAL NETWORK, DEVICE AND MEDIUM" and filed with the National Intellectual Property Administration, PRC on November 30, 2020, which is incorporated herein by reference in its entirety.

### FIELD OF THE TECHNOLOGY

This application relates to medical, artificial intelligence, cloud data and other technical fields, and in particular, to an omics data processing method and apparatus based on a graph neural network, a device and a medium.

### BACKGROUND OF THE DISCLOSURE

The gene expression and protein expression of the human body may differ greatly at different stages of life cycle and disease development. Therefore, omics (genomics, transcriptomics, proteomics and metabolomics, etc.) is an important tool for systematically studying the laws of biology. Moreover, because omics can also reflect the life cycle stage and disease development of the body, omics data plays a crucial role in health care.

### SUMMARY

Embodiments of this application provide a method for processing clinical omics data based on a graph neural network, the method including:
acquiring first omics data of a target object;
extracting at least two first omics features from the first omics data;
determining a first correlation between different omics features of the at least two first omics features;
constructing, based on the at least two first omics features and the first correlation, a first graph structure corresponding to the first omics data, the first graph structure including at least two nodes and at least one connecting edge, each node representing one of the first omics features in the first omics data, the at least one connecting edge connecting the at least two nodes, and representing a first correlation corresponding to two connected nodes;
detecting a node feature of each node in the first graph structure through a first graph neural network based on the first graph structure, the node feature corresponding to at least one feature category; and
analyzing the target object based on the detected node feature of each node to obtain an analysis result corresponding to each feature category of the at least one feature category; wherein the medical analysis comprises performing disease diagnosis, disease typing, and survival prediction on the target object; and the medical analysis results comprises a probability of the target object suffering from a disease corresponding to each feature category, a probability that the disease of the target object corresponding to each feature category is a certain disease category, and a survival probability of the target object corresponding to each feature category.

In another aspect, the embodiments of this application provide an apparatus for processing clinical omics data based on a graph neural network, the apparatus including:
a data acquisition module, configured to acquire first omics data of a target object, and extract at least two first omics features from the first omics data;
a correlation determination module, configured to determine a first correlation between different omics features of the at least two first omics features;
a graph structure construction module, configured to construct, based on the at least two first omics features and the first correlation, a first graph structure corresponding to the first omics data, the first graph structure including at least two nodes and at least one connecting edge, each node representing one of the first omics features in the first omics data, the at least one connecting edge connecting the at least two nodes, and representing a first correlation corresponding to two connected nodes;
a node feature determination module, configured to detect a node feature of each node in the first graph structure through a first graph neural network based on the first graph structure, the node feature corresponding to at least one feature category ; and
an analysis result determination module, configured to analyze medical analysis on the target object based on the detected node feature of each node to obtain an analysis result corresponding to each feature category in the at least one feature category; wherein the medical analysis comprises performing disease diagnosis, disease typing, and survival prediction on the target object; and the medical analysis results comprises a probability of the target object suffering from a disease corresponding to each feature category, a probability that the disease of the target object corresponding to each feature category is a certain disease category, and a survival probability of the target object corresponding to each feature category.

According to still another aspect, an embodiment of this application provides an electronic device, including a processor and a memory, the memory being configured to store a computer program, the computer program, when executed by the processor, causing the processor to perform the foregoing omics data processing method based on a graph neural network.

According to yet another aspect, an embodiment of this application provides a computer-readable storage medium, the computer-readable storage medium being configured to store a computer program, the computer program, when run on a computer, causing the computer to perform the foregoing omics data processing method based on a graph neural network.

### BRIEF DESCRIPTION OF THE DRAWINGS

To describe the technical solutions in embodiments of this application more clearly, the following briefly describes the accompanying drawings required for describing the embodiments of this application.
FIG. 1a is a schematic flowchart of an omics data processing method based on a graph neural network according to an embodiment of this application.
FIG. 1b is a specific flowchart of step S104 of obtaining a node feature of each node in a first graph structure through a first graph neural network based on the first graph structure according to an embodiment of this application.
FIG. 1c is a specific flowchart of obtaining, for each node in the first graph structure, a second feature of at least one hierarchy of the node according to an embodiment of this application.
FIG. 1d is a flowchart of a clinical omics data processing method based on a graph neural network according to an embodiment of this application.
FIG. 2a is a principle diagram of a clinical omics data processing method based on a graph neural network according to an embodiment of this application.
FIG. 2b is a schematic diagram of an edge matrix according to an embodiment of this application.
FIG. 3 is a schematic structural diagram of an omics data processing apparatus based on a graph neural network according to an embodiment of this application.
FIG. 4 is a schematic structural diagram of an electronic device according to an embodiment of this application.

### DESCRIPTION OF EMBODIMENTS

The following describes embodiments of this application in detail. Examples of the embodiments are shown in the accompanying drawings, and same or similar reference signs in all the accompanying drawings indicate same or similar components or components having same or similar functions. The embodiments that are described below with reference to the accompanying drawings are exemplary, and are only used to interpret this application and cannot be construed as a limitation to this application.

A person skilled in the art may understand that, the singular forms "a", "an", "said", and "the" used herein may include the plural forms as well, unless the context clearly indicates otherwise. It is to be further understood that, the terms "include" and/or "comprise" used in this specification of this application refer to the presence of stated features, integers, steps, operations, elements, and/or components, but do not preclude the presence or addition of one or more other features, integers, steps, operations, elements, components, and/or combinations thereof. It is to be understood that, when an element is "connected" or "coupled" to another element, the element may be directly connected to or coupled to another element, or an intermediate element may exist. In addition, the "connection" or "coupling" used herein may include a wireless connection or a wireless coupling. The term "and/or" used herein includes all of or any of units and all combinations of one or more related listed items.

With the vital role of omics data in health care, there are a number of statistical methods and machine learning methods that use omics data for the diagnosis, typing, and prediction of diseases. However, the idea of the statistical methods is focused on the analysis of differential proteins, which requires a large amount of manual intervention and cannot obtain clear classification or typing boundaries. An omics analysis method based on machine learning specifically includes: first acquiring sample omics features, and acquiring sample category tags of the sample omics features calibrated by a doctor, and then dividing all the sample omics features into a training set, a validation set, and a test set according to a certain proportion; and using sample omics features in the training set as an input, using corresponding sample category tags as supervision signals to train a model, and screening optimal parameters of the model according to the performance of the validation set to obtain a final model, and then performing disease prediction on the omics data based on the final model. However, the following shortcomings have been found in the omics data analysis method based on machine learning:
1. Since each disease development has its corresponding cascaded regulatory network, different features are correlated and regulated, but the machine model usually performs disease prediction based on each individual feature or a random combination of some features. It does not take into account the regulatory relationship naturally existing in these omics features, so it is impossible to explain the true pathogenic mechanism. The interpretability of the model is not strong, and the prediction accuracy is limited.
(2) Individual omics features or their random combinations are usually influenced by different experimental batches and experimental conditions, so the models obtained based on these features are more influenced by the data batches, and the generalization ability is weaker.
(3) Since the regulatory network in biology is a cascaded amplification mechanism, the difference in advanced regulatory factors between different categories of samples is not significant, and the regulated functional proteins are significantly different, but the machine model usually detects the significantly different functional proteins as biomarkers, and ignores the regulatory factors that are more clinically meaningful.
(4) Related machine learning is mainly focused on genomics and transcriptomics, but less attention is paid to proteomics. However, proteomics research has irreplaceable advantages for diagnosis, typing and prediction of diseases, and the neglect of proteomics research is a major obstacle to the realization of precision medicine.

In summary, the current research on omics data does not fully utilize the cascaded regulation relationship between the omics features, which cannot well reveal the real biological process of disease development. The interpretability and prediction accuracy of prediction results obtained based on the current method need to be improved.

On this basis, the embodiments of this application provide an omics data processing method and apparatus based on a graph neural network, a device and a medium, which are intended to resolve some or all of the technical problems described above. In the embodiments of this application, during obtaining to-be-processed omics data, the omics data may be processed based on the artificial intelligence technology to obtain the corresponding medical prediction results. Specifically, in the embodiments of this application, the feature of each omics feature in the to-be-processed omics data may be obtained based on the machine learning technology in the artificial intelligence technology, and then a final medical prediction result may be obtained based on the features of each omics feature.

Artificial intelligence (AI) is a theory, method, technology, and application system that uses a digital computer or a machine controlled by the digital computer to simulate, extend, and expand human intelligence, perceive an environment, obtain knowledge, and use knowledge to obtain an optimal result. In other words, AI is a comprehensive technology of computer sciences, attempts to understand essence of intelligence, and produces a new intelligent machine that can react in a manner similar to human intelligence. AI is to study design principles and implementation methods of various intelligent machines, to enable the machines to have functions of perception, reasoning, and decision-making.

The AI technology is a comprehensive discipline and relates to a wide range of fields including both hardware-level technologies and software-level technologies. Basic AI technologies generally include technologies such as a sensor, a dedicated AI chip, cloud computing, distributed storage, a big data processing technology, an operating/interaction system, and electromechanical integration. AI software technologies mainly include several major directions such as a computer vision (CV) technology, a speech processing technology, a natural language processing technology, and machine learning (ML)/deep learning.

Machine learning (ML) is a multi-field interdisciplinary subject involving the probability theory, statistics, the approximation theory, convex analysis, the algorithm complexity theory, and the like, The ML specializes in studying how a computer simulates or implements a human learning behavior to obtain new knowledge or skills, and reorganize an existing knowledge structure, so as to keep improving its performance. The ML is the core of the AI, is a basic way to make the computer intelligent, and is applied to various fields of AI. The ML and deep learning generally include technologies such as an artificial neural network, a belief network, reinforcement learning, transfer learning, inductive learning, and learning from demonstrations.

In some embodiments, the data processing/computing involved in the embodiments of this application may be performed in a cloud computing-based manner. Cloud computing refers to the delivery and use mode of IT infrastructures, and refers to obtaining the required resources in an on-demand and scalable manner through a network. Generalized cloud computing refers to the delivery and use mode of services, and refers to obtaining the required services in an on-demand and scalable manner through the network. Such services may be IT and software, Internet-related, or other services. Cloud computing is a product of the development and fusion of traditional computer and network technologies such as Grid Computing, Distributed Computing, Parallel Computing, Utility Computing, Network Storage Technologies, Virtualization and Load Balance.

With the diversified development of the Internet, real-time data flows and connected devices, as well as the demand for search services, social networks, mobile commerce and open collaboration, etc., cloud computing is developing rapidly. Unlike previous parallel distributed computing, the emergence of cloud computing will ideally drive revolutionary changes in the entire Internet mode and enterprise management mode.

The following describes the technical solutions of this application and how to resolve the foregoing technical issues according to the technical solutions of this application in detail by using specific embodiments.

First, several terms involved in this application are introduced and explained.

Omics: it is an important tool for systematically studying the laws of biology, mainly including genomics, proteomics, metabolomics, transcriptomics, lipidomics, immunomics, radiomics, and ultrasomics, etc. Omics feature: it is a relevant feature of various omics that may reflect the biology laws .

Biomarker: it refers to a biochemical indicator that may mark changes or possible changes in the structure or function of the systems, organs, tissues, cells, and subcells, and has a very wide range of uses, and may be used for disease diagnosis, for diagnosing disease staging, or for evaluating the safety and efficacy of new drugs or new therapies in a target population.

Signal pathway: it refers to the phenomenon that in a case that a certain reaction is to occur in a cell, a signal is transmitted from outside the cell to inside the cell, and the cell is to react according to this information. In the embodiments of this application, in a case that an omics feature interacts with other omics features in response to performing a function, the omics feature and other omics features may constitute a signal pathway.

The following describes the technical solutions of this application and how to resolve the foregoing technical issues according to the technical solutions of this application in detail by using specific embodiments. The following several specific embodiments may be combined with each other, and the same or similar concepts or processes may not be described repeatedly in some embodiments. The following describes the embodiments of this application with reference to the accompanying drawings.

In some embodiments, the method provided by the embodiments of this application is executed by an electronic device, which may be a server or a terminal device. Specifically, the method provided by the embodiments of this application may be executed based on data interactions between the terminal device or the server and the terminal device. The server may be an independent physical server, or may be a server cluster composed of a plurality of physical servers or a distributed system, and may further be a cloud server that provides cloud computing services. The terminal device may be a smartphone, a tablet computer, a notebook computer, a desktop computer, or the like, but is not limited thereto. The terminal device and the server may be directly or indirectly connected in a wired or wireless communication manner. This is not limited in this application. The method provided by the embodiments of this application is executed in response to performing data interaction based on the server and the terminal device, the terminal device may first transmit the to-be-processed omics data to the server, the server performs medical analysis on the received omics data, obtains a medical analysis result and returns it to the terminal device, and then the terminal device provides the medical analysis result to the user.

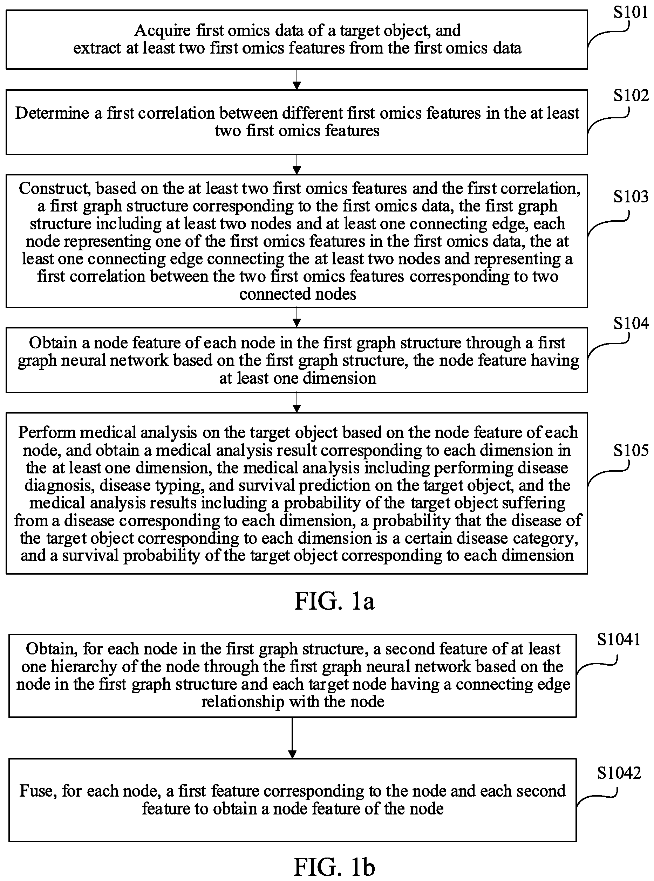

FIG. 1a illustrates a schematic flowchart of an omics data processing method based on a graph neural network according to an embodiment of this application. As shown in FIG. 1a, the method includes the following steps:
Step S101: Acquire first omics data of a target object, and extract at least two first omics features from the first omics data.

The first omics data of the target object refers to omics data that requires medical analysis, the first omics data includes at least two first omics features, the first omics features included belongs to the same category, e.g., all belong to genomics, and belongs to a same target object, but are different. In some embodiments, the category of the target object is not limited by the embodiments of this application, for example, the target object may be a human being, an animal, or the like. In an example, it is assumed that certain first omics data corresponds to genomics and includes genes 1 to 10 belonging to a person A. In this case, genes 1 to 10 are different genes.

Step S102: Determine a first correlation between different first omics features in the at least two first omics features.

In practical applications, different omics features are usually not accomplished independently in response to performing the function, but need to be accomplished jointly in combination with other omics features, that is, the omics features are correlated and have a regulatory relationship. On this basis, the first correlation between different first omics features may be determined in the embodiments of this application, and then the first omics features having similar functions may be associated based on the first correlation between the first omics features.

In response to determining the first correlation between different first omics features, a correlation matrix between different omics features may be calculated by Weighted Gene Co-Expression Network Analysis (WGCNA), and then binary processing may be performed on the correlation matrix by setting a threshold, and the correlation matrix subjected to the binary processing is called an edge matrix. For example, in a case that the correlation between two first omics features is not less than the threshold, it is indicated that the functions executed by the two first omics features are similar and interact with each other (i.e., constituting a signal pathway). In this case, values of elements in the correlation matrix representing the correlation between the two first omics features may be set to 1, and in a case that the correlation between the two first omics features is less than the threshold, it is indicated that the correlation between the two first omics features is lower, the values of the elements in the correlation matrix representing the correlation between the two first omics features may be set to 0.

In the embodiments of this application, the correlation between different first omics features is calculated in a WGCNA manner, and the first omics features having similar functions may have a higher correlation. Furthermore, after obtaining the correlation matrix between different first omics features, binary processing may be performed on the correlation matrix, to better highlight the correlation between the different first omics features.

Step S103: Construct, based on the at least two first omics features and the first correlation, a first graph structure corresponding to the first omics data, the first graph structure including at least two nodes and at least one connecting edge, each node representing one of the first omics features in the first omics data, the at least one connecting edge connecting the at least two nodes and representing a first correlation between the two first omics features between the two connected nodes.

Each node and the connecting edges of the nodes are included in the graph structure. In the embodiments of this application, each node in the graph structure represents a first omics feature, and the connecting edge between two nodes represents a first correlation between two first omics features corresponding to the two nodes. Accordingly, in response to constructing a first graph structure corresponding to the first omics data, each node included in the first graph structure may be obtained according to first omics features included in the first omics data, and then determine, according to the first correlation between different first omics features, that a connecting edge is specifically established between which two nodes in the first graph structure, to obtain the first graph structure corresponding to the first omics data.

In some embodiments of this application, the constructing, based on the at least two first omics features and each first correlation, a first graph structure corresponding to the first omics data includes:
establishing, for any two first omics features, a connecting edge between two nodes corresponding to the two first omics features in a case that a first correlation between the two first omics features is greater than or equal to a set value.

In some embodiments, during knowing the first correlation between different first omics features, for any two first omics features in the first omics data, in response to determining that the first correlation between any two first omics features is greater than or equal to the set value, it is indicated that the two first omics features execute similar functions and have a higher correlation with each other. In this case, a connecting edge may be established between the two nodes corresponding to the two first omics features in the first graph structure.

In some embodiments, in a case that the first correlation between different first omics features may be embodied based on the edge matrix described above, the first graph structure corresponding to the first omics data is constructed. For any two first omics features, in a case that the element value representing the first correlation between the two first omics features in the edge matrix is 1, a connecting edge may be established between two nodes corresponding to the two first omics features, and in a case that the element value representing the first correlation between the two first omics features in the edge matrix is 0, no connecting edge is established between two nodes corresponding to the two first omics features.

In the embodiments of this application, by constructing the graph structure of the omics data, the omics features that execute similar functions may be connected in the graph. In this case, not only the individual omics features may be reflected, but also the action relationship between different omics features may be reflected, and the pathogenic mechanism may be better revealed, and the simulation of biological processes may be realized, so that more accurate disease prediction effects may be obtained.

Step S104: Detect a node feature of each node in the first graph structure through a first graph neural network based on the first graph structure, the node feature corresponding to at least one feature category.

In some embodiments, the node feature may be a sequence or array in a case that the dimensions of the node feature are a plurality of dimensions.

The first graph neural network is a graph neural network corresponding to the omics to which the first omics data belongs, and the specific type of the first graph neural network may be pre-configured. For example, the graph neural network may be a Graph Attention Network (GAT), and other graph neural networks, such as a graph convolution network, a graph self-encoder network, etc., which is not limited in the embodiments of this application.

In some embodiments, in a case that the first graph structure corresponding to the first omics data is obtained, the feature of each first omics feature, i.e., the node feature of each node in the first graph structure, may be obtained through the graph neural network corresponding to the first omics data.

In some embodiments of this application, the method further includes:
extracting a first feature of each first omics feature.

FIG. 1b illustrates a specific flowchart of step S104 of obtaining a node feature of each node in the first graph structure through the first graph neural network based on the first graph structure. As shown in FIG. 1b, step S104 specifically includes the following steps:
Step S 1 041: Obtain, for each node in the first graph structure, a second feature of at least one hierarchy of the node through the first graph neural network based on the node in the first graph structure and each target node having a connecting edge relationship with the node.
Step S1042: Fuse, for each node, a first feature corresponding to the node and each second feature to obtain a node feature of the node.

In some embodiments, for each node in the first graph structure, a first feature for representing each node may be extracted (that is, a feature for representing each first omics feature may be extracted). For each node in the first graph structure, each target node having a connecting edge relationship with the node may be determined. Then, feature extraction is performed on the first feature of the node at least once through the first graph neural network based on the first feature of each target node and the first feature of the node, to obtain a second feature of at least one hierarchy of the node, the first feature being a feature of each node in the first graph structure that includes a single first omics feature during fusing with other features. In some embodiments, the first graph neural network may include at least one feature extraction layer (e.g., the GAT layer), and an output of each feature extraction layer corresponds to a second feature, where inputs of the first feature extraction layer is a first feature of each node in the first graph structure, and a connecting edge relationship between the nodes in the first graph structure, inputs of the feature extraction layers other than the first feature extraction layer are a second feature of each node corresponding to the previous feature extraction layer, and a connecting edge relationship between the nodes. The feature extraction layer may be a GAT layer based on an attention mechanism.

In some embodiments, in obtaining the first feature and the second feature corresponding to each node, for each node, the first feature and the at least one second feature corresponding to the node may be fused, the fused feature is used as a node feature of the node, and then medical analysis is performed based on the node feature of each node to obtain a corresponding medical analysis result.

In response to fusing the first feature and the at least one second feature of each node, the first feature and the at least one second feature may be mapped to a same node feature category through respective fully connected layers, to obtain each mapped feature, and then the mapped features are fused by stitching, and the fused feature is used as the node feature of each node.

In the embodiments of this application, since the second feature of each node is obtained based on the feature of the nodes constituting the signal pathway, the second feature of each node obtained in this case fuses the omics features of the other nodes (that is, the second feature is the feature of the signal pathway level). In this case, the node feature of each node obtained by fusing the first feature and the second feature of each node simultaneously includes a feature (i.e., the first feature) of a single omics feature level, and a feature (i.e., the second feature) of the signal pathway level, and may better represent the corresponding omics feature of the first omics data of the target object, so that the obtained analysis results may be more accurate in a case that medical analysis is performed based on the first omics data.

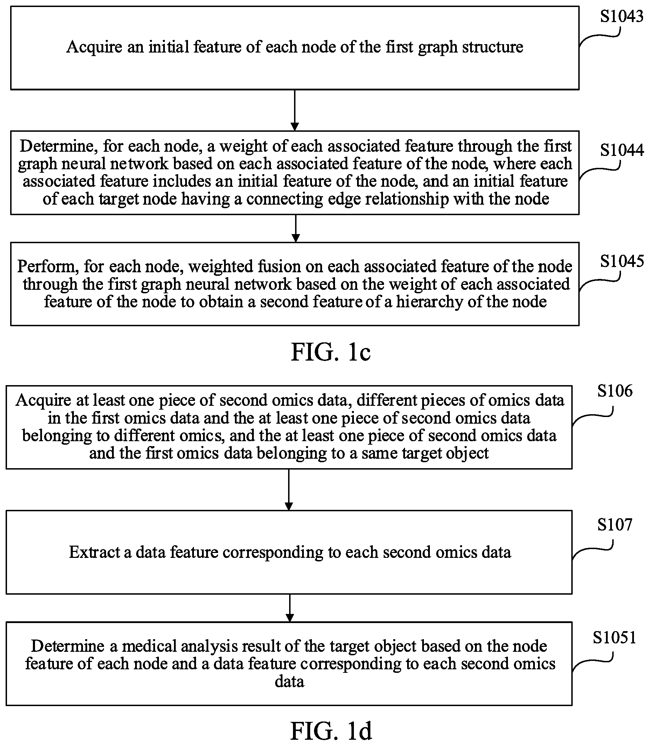

In the embodiments of this application, FIG. 1c illustrates a specific flowchart of obtaining, for each node in the first graph structure, a second feature of at least one hierarchy of the node through the first graph neural network based on the node in the first graph structure and each target node having a connecting edge relationship with the node in the embodiments of this application. As shown in FIG. 1c, the step includes the following steps:
Step S 1043 : Obtain an initial feature of each node of the first graph structure, where in response to determining a second feature of a first hierarchy of each node, the initial feature of each node is the first feature corresponding to each node, and in response to determining a second feature of any hierarchy other than the feature of the first hierarchy, the initial feature of each node is the second feature of a previous hierarchy of the hierarchy.
Step S 1044: Determine, for each node, a weight of each associated feature through the first graph neural network based on each associated feature of the node, where each associated feature includes an initial feature of the node, and an initial feature of each target node having a connecting edge relationship with the node.
Step S1045: Perform, for each node, weighted fusion on each associated feature of the node through the first graph neural network based on the weight of each associated feature of the node to obtain a second feature of a hierarchy of the node.

In a case that a node corresponds to a second feature of at least two hierarchies, the second feature of any hierarchy other than the second feature of the first hierarchy is obtained based on the second feature of the previous hierarchy of the hierarchy.

In some embodiments, for each node in the graph structure, an initial feature of each node may be determined, where in a case that a node corresponds to a second feature of at least two hierarchies, the second feature of any hierarchy other than the feature of the first hierarchy is obtained based on the second feature of the previous hierarchy of the hierarchy, that is, in response to determining the second feature of the first hierarchy of each node, the initial feature of each node is a first feature corresponding to each node, and in response to determining the second feature of any hierarchy other than the feature of the first hierarchy, the initial feature of each node is the second feature of the previous hierarchy of the hierarchy.

In actual applications, for any node, in a case that a connecting edge exists between the node and a certain node, it is indicated that the first omics feature corresponding to the node and a first omics feature corresponding to the certain node execute similar functions, and may constitute a signal pathway. However, the degree of importance of each node in the execution of the function is different. In this case, the degree of importance of each node in the execution of the function may be represented by a weight.

In some embodiments, for each node, each target node having a connecting edge to the node may be determined. Then, based on the initial feature of the node and the initial feature of each target node corresponding to the node (i.e., each associated feature of the node), the weight of each associated feature is determined through a graph convolution network (that is, the weights of the initial feature of the node and the initial feature of each target node corresponding to the node are determined). Then, the initial feature of the node and the initial feature of each target node may be weighted according to respective corresponding weights of the initial features of the node and the initial feature of each target node corresponding to the node, to obtain each weighted initial feature, and then the weighted initial features are fused, and the fused features are fused into a second feature of a hierarchy of the node.

In an example, it is assumed that the first graph neural network includes two GAT layers, the first graph structure includes three nodes (nodes 1 to 3), and connecting edges exist between the node 1 and the node 2, and between the node 1 and the node 3, respectively, and the node 2 and the node 3 only have a connecting edge to the node 1. In this case, the first features of the nodes 1 to 3 may be determined respectively. For the node 1, it may be determined that the target node of the node is the node 2 and the node 3. Then, based on the first feature of the node 1 (i.e., the initial feature of the node 1), and the first features corresponding to the nodes 2 and 3 (i.e., the initial features of the nodes 2 and 3), the weights of the first features of the nodes 1 to 3 may be determined through the first GAT layer in the graph convolution network. Then, the first features of the nodes 1 to 3 may be weighted and fused through the first GAT layer according to the respective weights of the first features of the nodes 1 to 3, respectively, to obtain the second feature of the first hierarchy of the node 1. Based on the same manner, the second features of the first hierarchies of the node 2 and the node 3 may be obtained. Furthermore, For the node 1, the second features of the first hierarchies of the nodes 1 to 3 may be used as the associated feature of the node 1, and then the weight of each associated feature may be determined through the graph convolution network. Then, based on the weight of each associated feature of the node, weighted fusion is performed on the associated feature of the node 1 through the second GAT layer of the graph convolution network to obtain a second feature of a second hierarchy of the node 1, and similarly, the second features of the second hierarchies of the node 2 and the node 3 may be obtained, respectively.

In the embodiments of this application, since the second feature of each node is obtained by weighted fusion of the feature of the node and the feature of a node connected thereto, it is possible to fuse the omics features of similar functions (the omics features constituting the signal pathway). The second feature obtained in this case is the feature of the signal pathway level, and it is possible to achieve more attention to advanced regulatory factors.

Step S105: Analyze the target object based on the detected node feature of each node to obtain an analysis result corresponding to each feature category in the at least one feature category, the medical analysis including performing disease diagnosis, disease typing, and survival prediction on the target object. The analysis results include a probability of the target object suffering from a disease corresponding to each feature category, a probability that the disease of the target object corresponding to each feature category is a certain disease category, and a survival probability of the target object corresponding to each feature category.

In some embodiments, in response to obtaining the node feature of each node, the medical analysis may be performed based on the feature of each node to obtain the corresponding medical analysis result. The categories specifically included in the medical analysis may be pre-configured, and the embodiments of this application are not limited, for example, at least one of disease recognition, disease typing, or survival prediction may be performed based on the node feature of each node. In this case, the resulting medical analysis result may include at least one of a disease recognition result, a disease typing result, or a survival prediction result.

In the embodiments of this application, for the to-be-processed omics data, each omics feature map in the omics data may be structured according to the correlation between different omics features, so that the correlation and regulatory relationship between the various omics features in biology may be effectively simulated, and the states of the omics features may be better represented. Accordingly, the node feature of each node in the graph structure may be obtained through the graph neural network based on the graph structure, and then the corresponding medical analysis result is obtained based on the node feature of each node. Since the structured omics features of the graph may effectively simulate the correlation and regulatory relationship between the biological omics features. In this case, the node feature of each node obtained through the graph neural network fuses the features of other nodes based on the graph structure, belongs to comprehensive features of the signal pathway level, and may reflect the correlation and regulatory relationship between the omics features, the represented content is richer, and the medical analysis results obtained based on the node features of the nodes may be more accurate.

In some embodiments of this application, FIG. 1d illustrates a flowchart of a clinical omics data processing method based on a graph neural network according to an embodiment of this application. As shown in FIG. 1d, the method further includes the following steps:
Step S106: Acquire at least one piece of second omics data, different pieces of omics data in the first omics data and the at least one piece of second omics data belonging to different omics, and the at least one piece of second omics data and the first omics data belonging to the same target object.
Step S107: Extract a data feature corresponding to each second omics data.

The obtaining a medical analysis result based on the node feature of each node includes the following steps:
Step S 1 051: Determine a medical analysis result of the target object based on the node feature of each node and a data feature corresponding to each second omics data.

Each second omics data and the first omics data belong to a same target object, and each second omics data and the first omics data belong to different categories of omics. For example, the first omics data is genomics, the second omics data is proteomics, and the second omics data and the first omics data belong to a person A.

In a case that the medical analysis result of the target object is determined jointly based on the data feature corresponding to each second omics data and the node feature of each node, the degrees of importance of the first omics data and each second omics data in determining the medical analysis result may be different, and in this case, the degrees of importance of the first omics data and each second omics data in determining the medical analysis result may be represented by setting different weights. Accordingly, in response to determining the medical analysis result, weighted fusion may be performed on the data feature corresponding to each second omics data and the node feature of each node based on the respective weights of the first omics data and each second omics data to obtain the fused feature, and then the medical analysis result of the target object may be determined based on the fused feature.

In the embodiments of this application, since other pieces of omics data belonging to a same target object but different category from the first omics data are also fused in response to determining the medical analysis result corresponding to the target object, a more comprehensive and accurate medical analysis is achieved, and the accuracy of the medical analysis result is improved.

In some embodiments of this application, each second omics data includes at least two second omics features.

The obtaining a node feature of each node in the first graph structure through a first graph neural network based on the first graph structure includes:
obtaining a node feature of each node in the first graph structure through a first graph neural network corresponding to the omics to which the first omics data belongs based on the first graph structure.

The extracting, for any second omics data, a data feature corresponding to the second omics data includes:
determining a second correlation between different second omics features in the at least two second omics features of the second omics data;
constructing, based on the at least two second omics features and each second correlation, a second graph structure corresponding to the second omics data; and
obtaining a node feature of each node corresponding to the second omics data through a second graph neural network corresponding to omics to which the second omics data belongs based on the second graph structure, to obtain the data feature corresponding to the second omics data, the data feature including a node feature of each node corresponding to the second omics data.

The second omics data includes at least two second omics features, and the second omics features and the first omics feature belong to different categories of omics. In some embodiments, the graph neural network to which each of the omics corresponds may be pre-configured, for example, the graph neural network to which the genomics corresponds, the graph neural network to which the proteomics corresponds, and the like may be pre-configured. Since the graph neural network to which each of the omics corresponds is trained based on different categories of sample omics features, in this case, the network parameters of the graph neural network to which each of the omics corresponds are different.

Accordingly, in response to obtaining the first omics data and the corresponding first graph structure, the node feature of each node of the first graph structure may be obtained based on the first graph neural network corresponding to the first omics to which the first omics data belongs. For any second omics data, a second correlation between different second omics features in the at least two second omics features included in the second omics data may be determined, and then a second graph structure corresponding to the second omics data may be constructed based on the second correlation between the different second omics features in the at least two second omics features. In this case, a node in the second graph structure represents a second omics feature, and a connecting edge in the second graph structure represents a second correlation between two second omics features corresponding to the two nodes of the connecting edge. Furthermore, the node feature of each node in the second graph structure, i.e., the data feature corresponding to the second omics data, may be obtained through the second graph neural network corresponding to the omics to which the second omics data belongs based on the second graph structure.

In some embodiments of this application, the obtaining a node feature of each node in the first graph structure through a first graph neural network based on the first graph structure, and the obtaining a medical analysis result based on the node feature of each node are obtained through an analysis result prediction model, where the analysis result prediction model is obtained by training an initial neural network model based on each sample omics data.

In some embodiments, each sample omics data and the initial neural network model may be obtained, and then the initial neural network model is trained based on the obtained sample omics data to obtain an analysis result prediction model. In this case, in response to determining the medical analysis result corresponding to to-be-processed first omics data, the first graph structure corresponding to the first omics data may be determined, and then the first graph structure corresponding to the first omics data may be inputted to the analysis result prediction model. The analysis result prediction model may obtain the node feature of each node in the first graph structure through the first graph neural network based on the first graph structure, and the medical analysis result is obtained and outputted based on the node feature of each node.

In some embodiments of this application, the analysis result prediction model is obtained in the following manners:
acquiring a training dataset and an initial neural network model, the training dataset including sample omics data and an annotation tag corresponding to each sample omics data, the annotation tag representing a real medical analysis results;
partitioning the training dataset into different sub-datasets;
iteratively training the initial neural network model based on different sub-datasets, respectively, until a preset training end condition is met; and
fusing model parameters of a corresponding neural network model at the end of each training, and using the fused model parameters as model parameters of the analysis result prediction model.

In some embodiments, in a case that the initial neural network model is trained based on each sample omics data, the training dataset and the initial neural network model may be obtained, where the training dataset includes each sample omics data and an annotation tag corresponding to each sample omics data, the annotation tag representing a real medical analysis result. Furthermore, the training dataset may be divided into different sub-datasets, and for each sub-dataset, iterative training is respectively performed on the initial neural network models based on the sub-dataset until the preset training end condition is met, to obtain an initial neural network model corresponding to the sub-dataset. Accordingly, in response to obtaining an initial neural network model corresponding to each sub-dataset, model parameters of the initial neural network model corresponding to each sub-dataset may be fused, and the fused model parameters are used as the model parameters of the analysis result prediction model.

In some embodiments, in practical applications, multiple pieces of sample omics data may be obtained at once, and then all sample omics data may be randomly divided into five subsets as five sub-datasets, one sub-dataset is taken as a test set each time, and the remaining four sub-datasets are taken as training sets for training the initial neural network model, and until the five sub-datasets are taken as the test set in turn, the initial neural network models corresponding to the five training maybe obtained. In this case, for each network parameter in the analysis result prediction model, average processing may be performed on the network parameter values in the initial neural network models corresponding to the five training, and the network parameter values subjected to the average processing may be used as the network parameter values of the analysis result prediction model, that is, the initial neural network model may be trained in a five-fold cross-validation mode to obtain the analysis result prediction model.

The training end condition may be the convergence of the value of a loss function corresponding to the initial neural network model, and the value of the loss function corresponding to the initial neural network model represents a difference between a predicted medical analysis result of the sample omics data and a real medical analysis result of the sample omics data. In a case that the value of the loss function converges, it is indicated that the accuracy of the current initial neural network model meets the requirements. In this case, the training may be ended.

In some embodiments, in a case that different types of medical analyses are required, output results of the analysis result prediction model are different, and the corresponding loss functions in response to training the analysis result prediction model are also different. For example, in a case that the category of medical analysis is disease diagnosis and disease typing, the predicted medical analysis result outputted by the initial neural network model is a prediction probability that the sample omics data corresponds to each category. In this case, the initial neural network model may be trained by minimizing a cross entropy between the prediction probability of all sample omics data and a tag of the medical analysis result. Moreover, in a case that the category of medical analysis is survival prediction, the predicted medical analysis result of the initial neural network model is a patient's risk coefficient. In this case, the initial neural network model may be trained by a loss function of cox (Cox proportional hazards model).

In practical applications, automated analysis may be performed on the omics data based on the analysis result prediction model provided in the embodiments of this application, thereby obtaining the early diagnosis and prediction results of the disease. In addition, since automated analysis is performed on the omics data by the analysis result prediction model provided in the embodiments of this application, the cascaded regulatory network in biology is simulated by performing structured processing on the graph of the omics data, so that the analysis result prediction model has higher interpretability and higher clinical applicability. Furthermore, compared with the traditional statistical test method and the analysis method based on human judgment, the final result may be automatically obtained, which saves time for analyzing omics data and waiting time, also effectively avoids manual judgment errors, and effectively improves the accuracy of medical analysis results.

In some embodiments of this application, the method further includes:
determining an importance parameter value corresponding to each first omics feature; and
providing the medical analysis result and the importance parameter value corresponding to each first omics feature to a user;
where the importance parameter value of each first omics feature is determined in the following manners:
   determining, for each sample omics data, based on the medical analysis result of the sample omics data, an importance parameter value of a node corresponding to each omics feature in the graph structure corresponding to the sample omics data; and
   obtaining, for any node, an importance parameter value of the node based on the importance parameter value of the node corresponding to all sample omics data, and using the importance parameter value of the node as an importance parameter value of an omics feature corresponding to the node.

The importance parameter value of the first omics feature represents the degree of importance of the first omics feature in a signal pathway constructed by the first omics feature. In some embodiments, in the embodiments of this application, the importance parameter values of each first omics feature may also be obtained, and the importance of each first omics feature and the obtained medical analysis result are provided to the user (e.g., provided to the medical personnel). In this case, the medical personnel may learn, according to the importance parameter value of each first omics feature, the omics feature that plays an important role in the medical analysis result, and then propose a biological explanation, which is conducive to enabling the patient to obtain more accurate medical treatment measures and achieve the purpose of accurate treatment.

In some embodiments, for the importance parameter value of each first omics feature, the importance parameter value of the first omics feature is the importance of the first omics feature in corresponding node in the first graph structure, and the importance of each node in the first graph structure may be determined based on the medical analysis result corresponding to the sample omics data, specifically:
during training to obtain an analysis result prediction model, for each sample omics data, a medical analysis result corresponding to the sample omics data may be obtained based on the analysis result prediction model, and in the process of obtaining a medical analysis result corresponding to the sample omics data, the sample omics data corresponds to each feature of each node in the graph structure (including a first feature and at least one second feature of at least one hierarchy). In this case, a gradient calculation (e.g., a derivative calculation) may be performed on each feature of each node based on the medical analysis result to obtain each calculated value, and then the calculated values are summed to obtain the importance parameter value of each node in the graph structure of the sample omics data, and based on the same method, the importance parameter value of each node in the graph structure of all sample omics data is obtained. It may be understood that since the number and category of sample omics features in each sample omics data are the same, the number of nodes in the graph structure of each sample omics data is the same, and the sample omics feature attributes represented by each node are the same. Accordingly, for any node in the graph structure, the importance parameter value of the node may be obtained based on the importance parameter value of the node in the graph structure of all sample omics data, and the importance parameter value of the node may be used as the importance parameter value of the corresponding omics feature of the node. For example, the importance parameter values of the nodes in the graph structure of all sample omics data may be summed, and the resulting sum value may be used as the importance parameter value of the node.

Furthermore, Important sample omics features may be determined based on the importance parameter value of each node, and the determined important sample omics features may then be enriched for the signal pathway (e.g., enrichment of signal pathways through a Metascape platform), to find the omics features that may be used as biomarkers at the signal pathway level.

In the embodiments of this application, the importance parameter value of the sample omics feature may be obtained by gradient calculation. In this case, not only the explanation and basis may be provided for the medical analysis result, but also the neural network model may be checked and corrected based on the importance parameter value of each sample omics feature. Moreover, it is also possible to obtain biomarkers that play an important role in disease prediction based on important sample omics features that may also be determined, so as to determine the disease prediction more accurately and determine the disease type.

In some embodiments of this application, the acquiring to-be-processed first omics data includes:
acquiring initial omics data, the initial omics data including at least two initial omics features;
acquiring an associated omics feature of the initial omics data, the associated omics feature and the initial omics data belonging to the same target object, and the associated omics feature including at least one of a case omics feature or an image omics feature; and
fusing each initial omics feature and the associated omics feature, respectively, to obtain a fusion omics feature corresponding to each initial omics feature, and using the fusion omics feature as a first omics feature.

The associated omics feature refers to a feature associated with the initial omics data, and the associated omics feature and the initial omics data belong to a same target object, and the specific category of the associated omics feature is not limited in this application. For example, the associated omics feature may include at least one of a case omics feature or an image omics feature of the target object.

In some embodiments, in response to acquiring the to-be-processed first omics data, at least two initial omics features and an associated omics feature belonging to the same target object as the initial omics data may be acquired, and then each initial omics feature and the associated omics feature are fused to obtain a fusion omics feature corresponding to each initial omics feature, and the fusion omics feature corresponding to each initial omics feature is used as the first omics feature included in the first omics data.

In the embodiments of this application, since the to-be-processed omics data used for determining the medical result fuses the omics feature of the target object and the associated feature of the omics feature, the feature expression of the to-be-processed omics data is richer in this case, thereby achieving more comprehensive and accurate medical analysis and improving the accuracy of the medical analysis result.

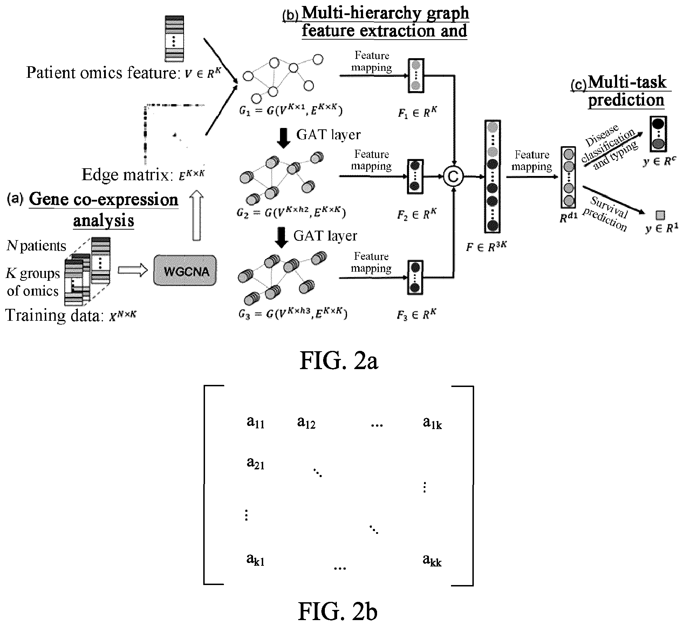

To better understand the method provided in the embodiments of this application, the method is described below in detail with reference to FIG. 2a. In this example, omics data of N patients may be obtained, and the omics data of each patient includes K different omics features (i.e., K groups of omics features in the graph). In this case, the omics data of N patients may be used as training data (i.e., the training data X^{N×K} in the figure) to train the initial graph neural network, to obtain an analysis result prediction model. Furthermore, medical analysis may be performed on the patient's omics data based on the analysis result prediction model, to obtain a final medical analysis result. In some embodiments, the method provided by embodiments of this application is described in detail in this example by taking determination of a medical analysis result corresponding to the omics data V ∈ R^{K} of one of the N patients (i.e., the to-be-processed first omics data including K different omics features), which may include:

In some embodiments, in response to determining the medical analysis result corresponding to the omics data of the patient, three parts may be included, i.e., (a) gene co-expression analysis, (b) multi-hierarchy graph feature extraction and fusion, and (c) multi-task prediction, where the multi-hierarchy graph feature extraction and fusion, and multi-task prediction may be implemented based on the analysis result prediction model obtained by training. In this case, the gene co-expression analysis part needs to be performed based on the omics data of the patient, and then the obtained result is inputted into the analysis result prediction model to obtain a final medical analysis result. Specifically, during acquiring the patient's omics data, a correlation matrix between different omics features may be calculated based on the weighted gene co-expression analysis technique (i.e. WGCNA), and then binary processing may be performed on the values of the elements in the correlation matrix by setting a threshold to obtain an edge matrix E^{K×K} with a feature category of K feature categories multiplied by K feature categories, and the edge matrix E^{K×K} includes elements aᵢⱼ (i=1, 2, ..., K, and j=1, 2, ..., K), specifically as shown in FIG. 2b. For example, for any elements a₁₂ in the correlation matrix, in a case that the degree of correlation between two omics features represented by the element a₁₂ is greater than a threshold, the value of the element a₁₂ is set to 1, otherwise set to 0.

Furthermore, by using each omics feature as a node, the connection between the nodes may be determined according to the edge matrix to obtain a graph structure corresponding to the omics data. For example, for any two omics features, in a case that the correlation between the two omics features is greater than or equal to a set value, a connecting edge is established between the two nodes corresponding to the two omics features. Feature extraction may then be performed based on the determined graph structure (e.g., feature extraction is performed by a fully connected layer, not shown in the figure) to obtain a first feature G1 of each node (a process of obtaining the first feature of each node is represented by G1=G1 (V^{K×1}, E^{K×K}) in the figure).

Furthermore, feature extraction may be performed on the first feature G1 of each node twice based on two GAT layers (i.e., the feature extraction layer in the foregoing text and the GAT layer in the figure), to obtain second features G2 and G3 of each node corresponding to two hierarchies. In response to determining G2, the first GAT layer may perform weighted summation on the first features of the connected nodes according to an attention value to obtain the second feature G2 of each node (a process of obtaining the second feature G2 of each node is represented by G2=G2 (V^{K×h2}, E^{K×K}) in the figure, h2 representing performing a second feature extraction), and the second GAT layer may perform weighted summation on the second features G2 of the connected nodes according to the attention value to obtain a second feature G3 of each node (a process of obtaining the second feature G3 of each node is represented by G3=G3 (V^{K×h3}, E^{K×K}) in the figure, h3 representing performing a second feature extraction). At this point, three different levels of features may be obtained for each omics feature, i.e., a local feature G1 (the feature of each node only includes a single omics feature) and overall features G2 and G3 (the feature of each node fuses the features of the omics features connected on the signal pathway).

Furthermore, the features G1, G2, and G3 of three levels may be mapped to features of a same feature category through the respective fully connected layers. For example, the feature during mapping of G1 is F₁ ∈ R^{K}, the feature during mapping of G2 is F₂ ∈ R^{K}, and the feature during mapping of G3 is F3 ∈ R^{K}. Then, the features F₁, F₂, and F₃ of the three levels may be fused by stitching to obtain the fused feature F ∈ R^{3K}, and then disease diagnosis, disease typing, or survival prediction may be performed based on F ∈ R^{3K}.

Further feature extraction (i.e., feature mapping) may be performed through a fully connected network during the disease diagnosis, disease typing, or survival prediction based on F ∈ R^{3K}, to obtain a feature R^{d1} (d1 represents that the feature category of feature R is d1 feature category). Disease diagnosis, disease typing, or survival prediction may then be performed based on the feature R^{d1} (i.e., (c) multi-task prediction part in the figure).

In some embodiments, in a case that disease diagnosis or disease typing (i.e., disease classification and typing in the figure) is performed based on the feature R^{d1}, the feature R^{d1} may be mapped to a feature with the feature category the same as the number of disease types or disease categories (c disease types or disease categories are taken as an example in this example), and then a disease prediction result or disease typing prediction result R^{c} (i.e., the medical analysis result in the foregoing text) is obtained based on the mapped feature. In this case, an output y of the analysis result prediction model is R^{c} (i.e., y ∈ R^{c}), where R^{c} represents a probability that the patient's omics data corresponds to each disease, or a probability that the patient's omics data corresponds to each category of diseases. In a case that survival prediction is performed based on the feature R^{d1} to determine a survival probability of the patient, the survival probability R¹ corresponding to the omics data of the patient (i.e., the medical analysis result in the previous text) may be obtained based on the feature R^{d1}. In this case, the output y of the analysis result prediction model is R¹ (i.e., y ∈ R¹).

Based on the description of the above embodiments, it can be seen that the method provided by the embodiments of this application simulates a cascaded regulatory network in biology by structuring the omics data graph, and then fully explores the effect of the association and interaction between the omics data on the disease development by using the graph neural network, and may fuse the graph structural features of different hierarchies, which not only extracts information of a single omics feature hierarchy, but also extracts the comprehensive features of the signal pathway hierarchy. Therefore, it may better represent the state of the data, so as to obtain a more accurate prediction result, and in response to determining the medical analysis result corresponding to the patient's omics data, it may be automatically performed based on the analysis result prediction model, and in this process, no manual intervention is required, which saves the analysis data and waiting time, and avoids the problems caused by artificial judgment errors. Compared with traditional technical solutions, it has obvious advantages, and may be implemented more intelligently and accurately in omics data analysis, thereby providing medical interventions more precisely to meet the actual needs of the medical personnel.

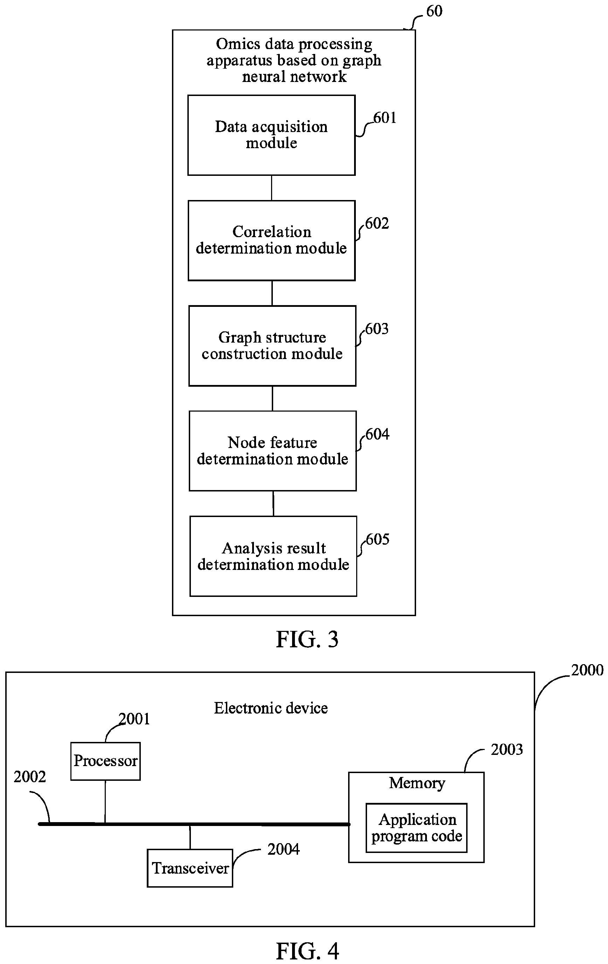

Embodiments of this application provide an omics data processing apparatus based on a graph neural network 60. As shown in FIG. 3, the omics data processing apparatus based on a graph neural network 60 may include: a data acquisition module 601, a correlation determination module 602, a graph structure construction module 603, a node feature determination module 604, and an analysis result determination module 605.

The data acquisition module 601 is configured to acquire first omics data of a target object, and extract at least two first omics features from the first omics data.

The correlation determination module 602 is configured to determine a first correlation between different omics features of the at least two first omics features.

The graph structure construction module 603 is configured to construct, based on the at least two first omics features and the first correlation, a first graph structure corresponding to the first omics data, the first graph structure including at least two nodes, and each node representing one of the first omics features in the first omics data, the first graph structure including at least one connecting edge that connects the at least two nodes, and the connecting edge representing a first correlation corresponding to two connected nodes.

The node feature determination module 604 is configured to obtain a node feature of each node in the first graph structure through a first graph neural network based on the first graph structure, the node feature having at least one feature category.

The analysis result determination module 605 is configured to perform medical analysis on the target object based on the node feature of each node, and obtain a medical analysis result corresponding to each feature category in the at least one feature category. The medical analysis including performing disease diagnosis, disease typing, and survival prediction on the target object. The medical analysis results including a probability of the target object suffering from a disease corresponding to each feature category, a probability that the disease of the target object corresponding to each feature category is a certain disease category, and a survival probability of the target object corresponding to each feature category.

In some embodiments, in response to constructing, based on the at least two first omics features and each first correlation, a first graph structure corresponding to the first omics data, the graph structure construction module is specifically configured to:
establish, for any two of the at least two first omics features, a connecting edge between two nodes corresponding to the two first omics features in a case that a first correlation between the two first omics features is greater than or equal to a set value, to construct the first graph structure.

In some embodiments, the apparatus further includes a feature extraction module and a node feature determination module. The feature extraction module is configured to:
extract, for each node in the first graph structure, a first feature of the first omics features, the first feature being a feature of each node in the first graph structure that includes only a single first omics feature.

In response to obtaining a node feature of each node in the first graph structure through a first graph neural network based on the first graph structure, the node feature determination module is specifically configured to:
obtain, for each node in the first graph structure, a second feature of at least one hierarchy of the node through the first graph neural network based on the node in the first graph structure and each target node having a connecting edge relationship with the node, each hierarchy corresponding to a feature extraction layer of the first graph neural network; and
fuse, for each node, a first feature corresponding to the node and each second feature to obtain a node feature of the node.

In some embodiments, in response to obtaining, for each node in the first graph structure, a second feature of at least one hierarchy of the node through the first graph neural network based on the node in the first graph structure and each target node having a connecting edge relationship with the node, the node feature determination module is specifically configured to:
acquire an initial feature of each node of the first graph structure;
determine, for each node, a weight of each associated feature through the first graph neural network based on each associated feature of the node, where each associated feature includes an initial feature of the node, and an initial feature of each target node connecting to the node;
perform, for each node, weighted fusion on each associated feature of the node through the first graph neural network based on the weight of each associated feature of the node to obtain a second feature of a hierarchy of the node.

In a case that a node corresponds to a second feature of at least two hierarchies, the second feature of any hierarchy other than the second feature of the first hierarchy is obtained based on the second feature of the previous hierarchy of the hierarchy.

In some embodiments, the feature extraction module is further configured to:
acquire at least one piece of second omics data, different pieces of omics data in the first omics data and the at least one piece of second omics data belonging to different omics, and the at least one piece of second omics data and the first omics data belonging to the same target object, and
extract a data feature corresponding to each second omics data.

In response to obtaining the medical analysis result based on the node features of each node, the analysis result determination module is specifically configured to:
determine a medical analysis result of the target object based on the node feature of each node and a data feature corresponding to each second omics data.

In some embodiments, each second omics data includes at least two second omics features.

In response to obtaining a node feature of each node in the first graph structure through a first graph neural network based on the first graph structure, the node feature determination module is specifically configured to:

In response to extracting, for any second omics data, a data feature corresponding to the second omics data, the feature extraction module is specifically configured to:
determine a second correlation between different second omics features in the at least two second omics features of the second omics data;
construct, based on the at least two second omics features and each second correlation, a second graph structure corresponding to the second omics data; and
obtain a node feature of each node corresponding to the second omics data through a second graph neural network corresponding to omics to which the second omics data belongs based on the second graph structure, to obtain the data feature corresponding to the second omics data, the data feature including a node feature of each node corresponding to the second omics data.

In some embodiments, the obtaining of a node feature of each node in the first graph structure through a first graph neural network based on the first graph structure, and the obtaining of a medical analysis result based on the node feature of each node are obtained through an analysis result prediction model, where the analysis result prediction model is obtained by training an initial neural network model based on each sample omics data.

In some embodiments, the apparatus further includes an information provision module, configured to:
acquire an importance parameter value corresponding to each first omics feature; and
provide the medical analysis result and the importance parameter value corresponding to each first omics feature to a user;
where the importance parameter value of each first omics feature is determined in the following manners:
   determining, for each sample omics data, based on the medical analysis result of the sample omics data, an importance of a node corresponding to each omics feature in the graph structure corresponding to the sample omics data; and
   obtaining, for any node, an importance parameter value of the node based on the importance of the node corresponding to all sample omics data, and using the importance parameter value of the node as an importance parameter value of an omics feature corresponding to the node.

In some embodiments, the analysis result prediction model is obtained in the following manners:
acquiring a training dataset and an initial neural network model, the training dataset including sample omics data and an annotation tag corresponding to each sample omics data, the annotation tag representing a real medical analysis results;
partitioning the training dataset into different sub-datasets;
iteratively training the initial neural network model based on different sub-datasets, respectively, until a preset training end condition is met; and
fusing model parameters of a corresponding neural network model at the end of each training, and using the fused model parameters as model parameters of the analysis result prediction model.

In some embodiments, in response to acquiring to-be-processed first omics data, the data acquisition module is specifically configured to:
acquire initial omics data, the initial omics data including at least two initial omics features;
acquire an associated omics feature of the initial omics data, the associated omics feature and the initial omics data belonging to the same target object, and the associated omics feature including at least one of a case omics feature or an image omics feature; and
fuse each initial omics feature and the associated omics feature, respectively, to obtain a fusion omics feature corresponding to each initial omics feature, and using the fusion omics feature as a first omics feature.

In some embodiments, the medical analysis result includes at least one of a disease recognition result, a disease typing result, or a survival prediction result.

The omics data processing apparatus based on a graph neural network in the embodiments of this application may execute an omics data processing method based on a graph neural network provided by the embodiments of this application, the implementation principle of which is similar, and is not repeated here.

The omics data processing apparatus based on a graph neural network may be a computer program (including a program code) running in a computer device, for example, the omics data processing apparatus based on a graph neural network is application software. The apparatus may be configured to execute corresponding steps in the method provided in the embodiments of this application.

In some other embodiments, the omics data processing apparatus based on a graph neural network provided in this embodiment of this application may be implemented in the form of a combination of software and hardware. For example, the omics data processing apparatus based on a graph neural network provided in this embodiment of this application may be a processor in a form of a hardware decoding processor, programmed to perform the omics data processing method based on a graph neural network provided in the embodiments of this application. For example, the processor in the form of a hardware decoding processor may use one or more application-specific integrated circuits (ASICs), DSPs, programmable logic devices (PLDs), complex programmable logic devices (CPLDs), field-programmable gate arrays (FPGAs), or other electronic components.

In other embodiments, the omics data processing apparatus based on a graph neural network provided by the embodiments of this application may be implemented in a software manner. FIG. 3 illustrates an omics data processing apparatus based on a graph neural network 60 stored in a memory, which may be software in the form of a program and plug-in, and includes a series of modules, including a data acquisition module 601, a correlation determination module 602, a graph structure construction module 603, a node feature determination module 604, and an analysis result determination module 605. The data acquisition module 601, the correlation determination module 602, the graph structure construction module 603, the node feature determination module 604, and the analysis result determination module 605 are configured to implement the omics data processing method based on a graph neural network provided by the embodiments of this application.

As shown in FIG. 4, an embodiment of this application provides an electronic device. An electronic device 2000 shown in FIG. 4 includes: a processor 2001 and a memory 2003. The processor 2001 and the memory 2003 are connected, for example, are connected by using a bus 2002. Optionally, the electronic device 2000 may further include a transceiver 2004. It is to be illustrated that during actual application, there may be one or more transceivers 2004. The structure of the electronic device 2000 does not constitute a limitation on this embodiment of this application.

The processor 2001 is applied in this embodiment of this application, and is configured to implement the functions of the modules shown in FIG. 3.

The processor 2001 may be a central processing unit (CPU), a general-purpose processor, a digital signal processor (DSP), an application-specific integrated circuit (ASIC), a field programmable gate array (FPGA) or another programmable logic device, a transistor logic device, a hardware component, or any combination thereof. The processor may implement or perform various examples of logic blocks, modules, and circuits described with reference to content disclosed in this application. The processor 2001 may be alternatively a combination to implement a computing function, for example, may be a combination of one or more microprocessors, or a combination of a DSP and a microprocessor.

The bus 2002 may include a channel, to transmit information between the foregoing components. The bus 2002 may be a PCI bus, an EISA bus, or the like. The bus 2002 may be classified into an address bus, a data bus, a control bus, and the like. For ease of description, the bus in FIG. 4 is represented by using only one bold line, but this does not indicate that there is only one bus or one type of bus.

The memory 2003 may be a ROM or another type of static storage device that can store static information and a computer program; or a RAM or another type of dynamic storage device that can store information and a computer program; or may be an EEPROM, a CD-ROM or another compact-disc storage medium, optical disc storage medium (including a compact disc, a laser disc, an optical disc, a digital versatile disc, a Blu-ray disc, or the like) and magnetic disk storage medium, another magnetic storage device, or any other medium that can be configured to carry or store an expected computer program in a form of a data structure and that is accessible by a computer, but is not limited thereto.

The memory 2003 is configured to store an application computer program for performing the solutions of this application, and is controlled and executed by the processor 2001. The processor 2001 is configured to execute the application computer program stored in the memory 2003, to implement the actions of the omics data processing apparatus based on a graph neural network provided in the embodiment shown in FIG. 3.

An embodiment of this application provides an electronic device, including a processor and a memory. The memory is configured to store a computer program, the computer program, when executed by the processor, causing the processor to perform any method in the above embodiments.

An embodiment of this application further provides a computer-readable storage medium. The computer-readable storage medium is configured to store a computer program, the computer program, when run on a computer, causing the computer to perform any method in the above embodiments.

According to an aspect of this application, a computer program product or a computer program is provided, the computer program product or a computer program including computer instructions, the computer instructions being stored in a computer-readable storage medium. A processor of a computer device reads the computer instructions from the computer-readable storage medium, and executes the computer instructions, so that the computer device performs the method provided in the foregoing various optional implementations.

The nouns and implementation principles involved in a computer-readable storage medium in this application may be specifically described with reference to an omics data processing method based on a graph neural network in the embodiments of this application, and are not repeated here.

It is to be understood that, although the steps in the flowchart in the accompanying drawings are sequentially shown according to indication of an arrow, the steps are not necessarily sequentially performed according to a sequence indicated by the arrow. Unless explicitly specified in this specification, execution of the steps is not strictly limited in the sequence, and the steps may be performed in other sequences. In addition, at least some steps in the flowcharts in the accompanying drawings may include a plurality of substeps or a plurality of stages. The substeps or the stages are not necessarily performed at the same moment, but may be performed at different moments. The substeps or the stages are not necessarily performed in sequence, but may be performed in turn or alternately with another step or at least some of substeps or stages of the another step.

The foregoing descriptions are some implementations of this application. A person of ordinary skill in the art may make several improvements and refinements without departing from the principle of this application, and the improvements and refinements shall fall within the protection scope of this application.

## Claims

1. A method for processing clinical omics data based on a graph neural network, carried out by an electronic device, the method comprising:
acquiring first omics data of a target object;
extracting at least two first omics features from the first omics data;
determining a first correlation between different omics features of the at least two first omics features;
constructing, based on the at least two first omics features and the first correlation, a first graph structure corresponding to the first omics data, the first graph structure comprising at least two nodes and at least one connecting edge, each node representing one of the first omics features in the first omics data, the at least one connecting edge connecting the at least two nodes and representing the first correlation between the two connected nodes;
detecting a node feature of each node in the first graph structure through a first graph neural network based on the first graph structure, the node feature corresponding to at least one feature category; and
analyzing the target object based on the detected node feature of each node to obtain an analysis result corresponding to each feature category of the at least one feature category;the medical analysis comprising performing disease diagnosis, disease typing, and survival prediction on the target object; and the medical analysis results comprising a probability of the target object suffering from a disease corresponding to each dimension, a probability that the disease of the target object corresponding to each dimension is a certain disease category, and a survival probability of the target object corresponding to each dimension.

2. The method according to claim 1, wherein the constructing, based on the at least two first omics features and the first correlation, the first graph structure corresponding to the first omics data comprises:
for any two of the at least two first omics features, in response to a first correlation between the two first omics features being greater than or equal to a set value, establishing a connecting edge between two nodes corresponding to the two first omics features to construct the first graph structure.

3. The method according to claim 1, wherein the method further comprises:
extracting, for each node in the first graph structure, a first feature of the first omics features, the first feature being a feature of each node in the first graph structure that comprises only a single first omics feature; and
the obtaining the node feature of each node in the first graph structure through thefirst graph neural network based on the first graph structure comprises:
obtaining, for each node in the first graph structure, a second feature of at least one hierarchy of the node through the first graph neural network based on the node in the first graph structure and each target node having a connecting edge relationship with the node, each hierarchy corresponding to a feature extraction layer of the first graph neural network; and
fusing, for each node, a first feature corresponding to the node and each second feature to obtain a node feature of the node.

4. The method according to claim 3, wherein the obtaining, for each node in the first graph structure, the second feature of at least one hierarchy of the node through the first graph neural network based on the node in the first graph structure and each target node having the connecting edge relationship with the node comprises:
acquiring an initial feature of each node of the first graph structure;
determining, for each node, a weight of each associated feature through the first graph neural network based on each associated feature of the node, wherein each associated feature of the node comprises an initial feature of the node, and an initial feature of each target node having a connecting edge relationship with the node; and
performing, for each node, weighted fusion on each associated feature of the node through the first graph neural network based on the weight of each associated feature of the node to obtain a second feature of a hierarchy of the node; wherein
in response to a node corresponding to second features of at least two hierarchies, a second feature of any hierarchy other than a first hierarchy is obtained based on a second feature of a previous hierarchy of the hierarchy.

5. The method according to claim 4, wherein the acquiring the initial feature of each node of the first graph structure comprises:
using, in response to determining a second feature of a first hierarchy of each node, the first feature corresponding to each node as the initial feature of each node; and
using, in response to determining a second feature of any hierarchy other than the first hierarchy, the second feature of the previous hierarchy of the hierarchies the initial feature of each node.

6. The method according to claim 1, the method further comprises:
acquiring at least one piece of second omics data, different pieces of omics data in the first omics data and the at least one piece of second omics data belonging to different omics, and the at least one piece of second omics data and the first omics data belonging to the same target object;
extracting a data feature corresponding to each second omics data;
the obtaining a medical analysis result based on the node feature of each node comprises:
determining the medical analysis result on the target object based on the node feature of each node and the data feature corresponding to each second omics data.

7. The method according to claim 6, wherein each second omics data comprises at least two second omics features; and
the extracting, for any second omics data, the data feature corresponding to the second omics data comprises:
determining a second correlation between different second omics features in the at least two second omics features of the second omics data;
constructing, based on the at least two second omics features and each second correlation, a second graph structure corresponding to the second omics data; and
obtaining a node feature of each node corresponding to the second omics data through a second graph neural network corresponding to omics to which the second omics data belongs based on the second graph structure, to obtain the data feature corresponding to the second omics data, the data feature comprising a node feature of each node corresponding to the second omics data.

8. The method according to claim 1, wherein the node feature of each node in the first graph structure obtained through the first graph neural network based on the first graph structure, and the medical analysis result obtained based on the node feature of each node are obtained through the analysis result prediction model, wherein the analysis result prediction model is obtained by training an initial neural network model based on each sample omics data.

9. The method according to claim 8, wherein the method further comprises:
determining an importance parameter value corresponding to each first omics feature; and
providing the medical analysis result and the importance parameter value corresponding to each first omics feature to a user;
wherein the importance parameter value of each first omics feature is determined in the following manners:
determining, for each sample omics data, based on the medical analysis result of the sample omics data, an importance of a node corresponding to each omics feature in the graph structure corresponding to the sample omics data; and
obtaining, for any node, an importance parameter value of the node based on the importance of the node corresponding to all sample omics data, and using the importance parameter value of the node as an importance parameter value of an omics feature corresponding to the node.

10. The method according to claim 8, wherein the analysis result prediction model is obtained in the following manners:
acquiring a training dataset and an initial neural network model, the training dataset comprising sample omics data and an annotation tag corresponding to each sample omics data, the annotation tag representing a real medical analysis results;
partitioning the training dataset into different sub-datasets;
iteratively training the initial neural network model based on different sub-datasets, respectively, until a preset training end condition is met; and
fusing model parameters of a corresponding neural network model at the end of each training, and using the fused model parameters as model parameters of the analysis result prediction model.

11. The method according to claim 1, wherein the acquiring first omics data of a target object comprises:
acquiring initial omics data of the target object, the initial omics data comprising at least two initial omics features;
acquiring an associated omics feature of the initial omics data, the associated omics feature and the initial omics data belonging to the same target object, and the associated omics feature comprising at least one of a case omics feature or an image omics feature; and
fusing each initial omics feature and the associated omics feature, respectively, to obtain a fusion omics feature corresponding to each initial omics feature, and using the fusion omics feature as a first omics feature.

12. An apparatus for processing clinical omics data based on a graph neural network, comprising:
a data acquisition module, configured to acquire first omics data of a target object, and extract at least two first omics features from the first omics data;
a correlation determination module, configured to determine a first correlation between different omics features of the at least two first omics features;
a graph structure construction module, configured to construct, based on the at least two first omics features and the first correlation, a first graph structure corresponding to the first omics data, the first graph structure comprising at least two nodes and at least one connecting edge, each node representing one of the first omics features in the first omics data, the at least one connecting edge connecting the at least two nodes, and representing the first correlation between the two connected nodes;
a node feature determination module, configured to detect a node feature of each node in the first graph structure through a first graph neural network based on the first graph structure, the node feature corresponding to at least one feature category; and
an analysis result determination module, configured to analyze medical analysis on the target object based on the detected node feature of each node to obtain an analysis result corresponding to each feature category in the at least one feature category;wherein: the medical analysis comprising performing disease diagnosis, disease typing, and survival prediction on the target object; and the medical analysis results comprising a probability of the target object suffering from a disease corresponding to each feature category, a probability that the disease of the target object corresponding to each feature category is a certain disease category, and a survival probability of the target object corresponding to each feature category.

13. An electronic device, comprising a processor and a memory,
the memory being configured to store a computer program, the computer program, when executed by the processor, causing the processor to perform the method according to any one of claims 1 to 11.

14. A computer-readable storage medium, the computer-readable storage medium being configured to store a computer program, the computer program, when run on a computer, causing the computer to perform the method according to any one of claims 1 to 11.

15. A computer program product, the computer program product comprising computer instructions, the computer instructions being stored in a computer-readable storage medium, a processor of a computer device reading the computer instructions from the computer-readable storage medium, and the processor executing the computer instructions to cause the computer device to perform the method according to any one of claims 1 to 11.
